# EUROPEAN PATENT APPLICATION

(11) **EP 4 332 220 A1**
(43) Date of publication of application: **06.03.2024**
(21) Application number: 22775735.8
(22) Date of filing: 24.03.2022
(51) Int. Cl.: C12N 15/11, C12Q 1/6876, C12Q 1/689

(54) **METHOD FOR AMPLIFYING TARGET GENE HAVING SPECIFIC GC CONTENT**

(30) Priority: 24.03.2021 JP 2021049853
(71) Applicant: Denka Company Limited, Tokyo 103-8338 (JP); Toho University, Tokyo, 143-8541 (JP)
(72) Inventor: TATEDA, Kazuhiro, Tokyo 143-8541 (JP); MIYATAKE, Yuya, Tokyo 103-8338 (JP)
(74) Representative: Mewburn Ellis LLP
(86) International application number: PCT/JP2022/013805
(87) International publication number: WO 2022/202954

(57) **Abstract**

The present invention provides a method for amplifying, in a reaction solution, at least one target gene having a GC content of at least 60%, wherein the method contains a step of amplifying a specific base sequence in a target gene by using a primer set consisting of a first primer and a second primer for target gene amplification, and wherein one primer in the primer set is added to the reaction solution at a molar ratio higher than the other primer and neither primer is a hot start primer that contains an inactivating chemical modification that is restored by an action of an activating enzyme.

## Description

### Technical Field

The present invention generally relates to, for instance, a method for amplifying, in a reaction solution, a target gene having a specific GC content by using asymmetric PCR.

*Pseudomonas aeruginosa* is a resident bacterium present on the epithelium and in the intestinal tract of humans and is a typical bacterial species associated with opportunistic infections. Multidrug-resistant *Pseudomonas aeruginosa,* having acquired drug resistance, is a frequent cause of nosocomial infection outbreaks. *Pseudomonas aeruginosa* is thus a bacterial species that is drawing the attention of medical institutions.

### Citation List

### Patent Document

Patent Document 1: Japanese Unexamined Patent Application Publication No. 2015-536653

### Summary

### Technical Problem

It is generally known that the genes carried by an organism have a species-specific constituent base composition and GC content. *Pseudomonas aeruginosa* is a bacterium characterized by a high GC content; for example, the result of an analysis of the complete genome of the PA01 strain was a GC content of 66.6% (Stover, C. K., Pham, X. Q., Erwin, A. L., Mizoguchi, S. D., Warrener, P., Hickey, M. J., Brinkman, F. S., Hufnagle, W. O., Kowalik, D. J., Lagrou, M. et al. 2000. Complete genome sequence of Pseudomonas aeruginosa PA01, an opportunistic pathogen. Nature 406: 959-964.).

However, it has been found that, when a bacterium having high GC content genes, such as *Pseudomonas aeruginosa,* is a target, the amount of synthesis of the PCR product of the target gene declines and the detection sensitivity declines. The decline in detection sensitivity becomes a particular problem for methods in which a target gene is to be amplified at the same time as amplification of a gene in a sample that contains various bacterial species to be differentiated in addition to the target gene and the target bacterial species that contains the same. Such a simultaneous amplification of different genes is frequently referred to as multiplex PCR, and is a procedure that can amplify a plurality of gene fragments from a single template DNA in a single reaction system. This multiplex PCR, which exhibits an excellent throughput, can also amplify respective gene fragments from a plurality of template DNAs present in a reaction system and has become an indispensable technique among genetic diagnostic technologies.

### Solution to Problem

**As** a result of extensive and intensive research in order to solve the aforementioned problem, the present inventors, by combining a technology for detecting a high GC content target gene (also referred to as the gene of interest (GOI)) with asymmetric PCR, created a comprehensive, highly sensitive, and highly specific technology that can simultaneously detect GOIs having a high GC content of at least 60%, as well as target genes not having a high GC content (GC content: 40 to 60%), and thus came to achieve the present invention.

That is, the present application comprises the following inventions.
[1] A method for amplifying, in a reaction solution, at least one target gene having a GC content of at least 60%, the method including a step of amplifying a specific base sequence in a target gene by using a primer set consisting of a first primer and a second primer target gene amplification, and wherein one primer in the primer set is added to the reaction solution at a molar ratio higher than the other primer and neither primer is a hot start primer that contains an inactivating chemical modification that is restored by an action of an activating enzyme.
[2] The method according to [1], wherein the reaction solution contains one or a plurality of genes that are not a target gene and wherein the method includes simultaneous amplification of both a target gene and the one or a plurality of genes that are not a target gene.
[3] The method according to [1] or [2], wherein the molar ratio of the primers in the primer set is 1 : 1.5 to 20.0.
[4] The method according to any of [1] to [3], wherein a target gene is a base sequence consisting of at least 13 consecutive bases in the genomic sequence of *Pseudomonas aeruginosa.*
[5] The method according to any of [1] to [4], wherein the first primer is a primer that hybridizes, under stringent conditions, with a base sequence described in any of SEQ ID NOs: 1 to 3 or with a base sequence provided by deletion, substitution, insertion, or addition of one or a plurality of bases in a base sequence described in any of SEQ ID NOs: 1 to 3, and the second primer is a primer that hybridizes, under stringent conditions, with a complementary strand sequence for a base sequence described in any of SEQ ID NOs: 1 to 3 or with a base sequence provided by the deletion, substitution, insertion, or addition of one or a plurality of bases in a complementary strand sequence for a base sequence described in any of SEQ ID NOs: 1 to 3.
[6] The method according to any of [1] to [5], wherein the first primer is an oligonucleotide consisting of at least 13 consecutive bases in the base sequence described in SEQ ID NO: 4 or in a base sequence provided by the deletion, substitution, insertion, or addition of one or a plurality of bases in the base sequence described in SEQ ID NO: 4.
[7] The method according to any of [1] to [6], wherein the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.
[8] The method according to any of [1] to [7], wherein a primer is labeled with biotin.
[9] The method according to any of [1] to [8], wherein the primer with a higher molar ratio is labeled with biotin.
[10] A method of detecting, in one reaction solution, at least one target gene having a GC content greater than 60%, the method including a step of detecting a target gene by using a probe that is complementary to a single strand of a target gene that has been amplified by the method described in any of [1] to [9].
[11] The method according to [10], wherein the probe is complementary to a single strand of the target gene that has been amplified by the primer with the higher molar ratio.
[12] The method according to [10] or [11], wherein a target gene is detected through occurrence of complementary double-stranded gene formation from the probe and a single strand of the target gene.
[13] The method according to any of [1] to [12], wherein a target gene originates from Pseudomonas aeruginosa and a bacterial gene from other than *Pseudomonas aeruginosa* is differentiated from *Pseudomonas aeruginosa.*
[14] The method according to any of [10] to [13], wherein the probe is immobilized on a detection substrate material.
[15] The method according to any of [10] to [14], wherein the probe is labeled.
[16] The method according to any of [10] to [15], wherein a probe nucleic acid contains an oligonucleotide consisting of at least 13 consecutive bases in the base sequence described in SEQ ID NO: 6 or in a base sequence provided by the deletion, substitution, insertion, or addition of one or a plurality of bases in the base sequence described in SEQ ID NO: 6.
[17] The method according to any of [14] to [16], wherein a detection substrate material is a coded microcarrier.
[18] The method according to [17], wherein the microcarrier contains:
   (a) a layer that is a substantially transparent polymer layer having a first surface and a second surface, with the first and second surfaces being parallel to each other;
   (b) a layer that is a substantially opaque polymer layer, this substantially opaque polymer layer being affixed to the first surface of the substantially transparent polymer layer and encompassing a central region of the substantially transparent polymer layer, and the substantially opaque polymer layer containing a two-dimensional shape that represents an analog code; and
   (c) a capture agent for capturing an analyte, the capture agent being attached to at least one of the first surface and the second surface of the substantially transparent polymer layer, in at least the central region of the substantially transparent polymer layer.
[19] A primer set, consisting of a first primer and a second primer, for detecting the presence of at least one target gene having a GC content of at least 60%, wherein the first primer is an oligonucleotide consisting of at least 13 consecutive bases in the base sequence described in SEQ ID NO: 4 or in a base sequence provided by deletion, substitution, insertion, or addition of one or a plurality of bases in the base sequence described in SEQ ID NO: 4, and the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.
[20] The primer set according to [19], wherein a target gene is a base sequence consisting of at least 13 consecutive bases in the genomic sequence of *Pseudomonas aeruginosa.*
[21] The primer set according to [19] or [20], wherein the first primer is a primer that hybridizes, under stringent conditions, with a base sequence described in any of SEQ ID NOs: 1 to 3 or with a base sequence provided by the deletion, substitution, insertion, or addition of one or a plurality of bases in a base sequence described in any of SEQ ID NOs: 1 to 3, and the second primer is a primer that hybridizes, under stringent conditions, with a complementary strand sequence for a base sequence described in any of SEQ ID NOs: 1 to 3 or with a base sequence provided by the deletion, substitution, insertion, or addition of one or a plurality of bases in a complementary strand sequence for a base sequence described in any of SEQ ID NOs: 1 to 3.
[22] The primer set according to any of [19] to [21], wherein the first primer is an oligonucleotide consisting of at least 13 consecutive bases in the base sequence described in SEQ ID NO: 4 or in a base sequence provided by the deletion, substitution, insertion, or addition of one or a plurality of bases in the base sequence described in SEQ ID NO: 4.
[23] The primer set according to any of [19] to [22], wherein the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.

### Advantageous Effects of Invention

**The** present invention enables the highly sensitive detection of high GC content genes through the use of asymmetric PCR.

### Brief Description of Drawings

Fig. 1 shows the results of multiple sequence alignment for the oprL gene of the E6130952 strain, NCTC10332 strain, and PAO1 strain of *P*. *aeruginosa* (SEQ ID NOs: 1, 2, 3).
Fig. 2 shows the results of a comparison of the fluorescence values in normal PCR and asymmetric PCR carried out on a template of genomic DNA extracted from *P. aeruginosa* strain JCM14847.
Fig. 3 shows the results for the sensitization factor for the amplification, using asymmetric PCR, of templates having GC contents different from that of genomic DNA extracted from *P. aeruginosa* strain JCM14847.

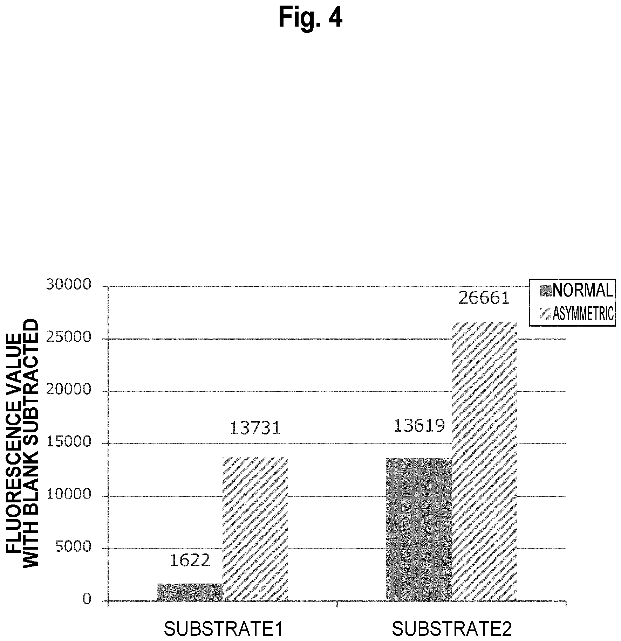

Fig. 4 shows the results of a comparison of the fluorescence values in normal PCR and asymmetric PCR carried out using as the template genomic DNA extracted from a culture medium that contained *P. aeruginosa* and *Enterococcus casseliflavus.*

### Description of Embodiments

Embodiments of the present invention (referred to hereafter as "the present embodiments") are described in the following, but this should not be construed as limiting the scope of the present invention to the following embodiments.

### (Gene amplification · detection methods)

A first aspect provides a method for amplifying, in a reaction solution, at least one target gene having a GC content of at least 60%. This amplification method can contain a step of amplifying a specific base sequence in a target gene using a primer set containing a first primer and a second primer for target gene amplification, and can contain other optional steps. The amplification step includes a step of bringing the primer set into contact with a target gene. The reaction solution may contain one or a plurality of genes that are not a target gene, in which case the target gene(s) and the one or a plurality of non-target genes are simultaneously submitted to the amplification reaction.

The target gene, also referred to as a gene of interest (GOI), may be any gene that has a GC content of at least 60%. When the amplification reaction is carried out using multiplex PCR, a target gene, among the plurality of genes that are to be amplified, is preferably a gene having a relatively high GC content. The GC content is the proportion (%) for the G base and C base in the DNA strand constituting the target gene and is determined by performing sequence analysis of the target gene. In addition to *Pseudomonas aeruginosa,* bacteria having a target gene with at least 60% can be exemplified by *Mycobacterium tuberculosis* (65.86%), *Propionibacterium acnes* (60.3%), and *Bifidobacterium* spp. *(Bifidobacterium longum* at 60.84% and *Bifidobacterium animalis* at 61.36%), but there is no limitation to these bacteria. The oprL gene (GC content: 62.3%) from *Pseudomonas aeruginosa* is an example of a target gene. The oprL gene is a gene in *Pseudomonas aeruginosa* that codes for a precursor to peptidoglycan lipoprotein.

The target gene amplification step is carried out using asymmetric PCR under amplification conditions. For example, the molar ratio of the primers added to the reaction solution for target gene amplification is managed so one primer has a higher concentration than the other primer. Japanese Unexamined Patent Application Publication No. 2015-536653 (supra) discloses, for the primers used in asymmetric PCR, hot start primers that contain an inactivating chemical modification that is restored by the action of an activating enzyme, but the primer set used in the present gene amplification method does not contain an inactivating chemical modification that is restored by the action of an activating enzyme, as in such hot start primers (riboprimers). On the other hand, with regard to the primers used to amplify a gene that is not a target gene, these may be any primers and the added amounts of the forward primer and reverse primer may also be an equal molar ratio.

The molar ratio of the primers for target gene amplification should be able to efficiently amplify a target gene in comparison to normal PCR, but is not otherwise particularly limited; however, for example, it is adjusted as appropriate within the range of 1 : 1.5 to 20.0 in the reaction solution prior to amplification. From the standpoint of further increasing the fluorescence intensity, the molar ratio is preferably 1 : 1.5 to 3.5, more preferably 1.5 to 3.0, and still more preferably 1.5 to 2.5. For the case of target gene amplification under asymmetric PCR conditions, the single strand that is the amplification product of the primer added in excess is produced more abundantly than the single strand that is amplified by the other primer, and as a consequence the efficiency of formation of the complementary strand between this single strand and the probe is also increased. A plurality of target genes may be present, in which case it becomes necessary to prepare a different primer set for each different target gene.

With regard to the design of the primer sequence for target gene amplification, the individual skilled in the art can, referring to the sequence of the target gene to be amplified, make a determination as appropriate of an oligonucleotide sequence that will be complementary. The primers used in the asymmetric PCR preferably are produced so the Tm values of the forward primer and reverse primer are on the same level. For example, when a target gene is a gene from *Pseudomonas aeruginosa,* primers are synthesized that are complementary to a base sequence composed of at least 13 consecutive bases in the genomic sequence of *Pseudomonas aeruginosa.* The oprL gene, with a GC content of approximately 62%, is an example of a target gene in Pseudomonas aeruginosa. The oprL gene is one of the genes that encode outer membrane proteins, and its sequence differs from strain to strain. For example, the oprL genes for *Pseudomonas aeruginosa* strain E6130952, strain NCTC10332, and strain PAO1 have the base sequences given in SEQ ID NOs: 1, 2, and 3, respectively.

The oprL gene has the base sequence given in any of SEQ ID NOs: 1 to 3 or has a base sequence provided by the deletion, substitution, insertion, or addition of one or a plurality of bases in a base sequence given in any of SEQ ID NOs: 1 to 3, and encodes OprL protein.

Functioning as the forward primer, the first primer may have any sequence that is able to hybridize, under stringent conditions, with a base sequence described in any of SEQ ID NOs: 1 to 3 or with a base sequence provided by the deletion, substitution, insertion, or addition of one or a plurality of bases in a base sequence described in any of SEQ ID NOs: 1 to 3.

Functioning as the reverse primer, the second primer may have any sequence that is able to hybridize, under stringent conditions, with a complementary strand sequence for a base sequence described in any of SEQ ID NOs: 1 to 3 or with a base sequence provided by the deletion, substitution, insertion, or addition of one or a plurality of bases in a complementary strand sequence for a base sequence described in any of SEQ ID NOs: 1 to 3.

**As** used in the present Description, "stringent conditions" denotes conditions in which a so-called specific hybrid is formed and nonspecific hybrids are not formed, and, for example, can be determined as appropriate based on data such as, e.g., the length of the base sequence. For example, stringent conditions can be established with reference to Molecular Cloning (Fourth Edition, Cold Spring Harbor Laboratory Press, New York). Stringent conditions may also refer to conditions in which highly homologous (preferably identical) polynucleotides, for example, polynucleotides having a homology of at least 70%, preferably at least 80%, more preferably at least 90%, still more preferably at least 95%, and particularly preferably at least 99%, hybridize with each other, and polynucleotides that exhibit a homology lower than this do not hybridize with each other.

The following conditions are specific stringent conditions: incubation at approximately 42°C to approximately 50°C in a hybridization solution (50% formamide, 10x SSC (0.15 M NaCl, 15 mM sodium citrate, pH 7.0), 5x Denhardt's solution, 1% SDS, 10% dextran sulfate, 10 µg/mL denatured salmon sperm DNA, and 50 mM phosphate buffer (pH 7.5)) of a base sequence complementary to a desired base sequence with a sample for which the presence of the target gene or bacterial species having the target gene is suspected, and washing at approximately 65°C to approximately 70°C using 0.1x SSC and 0.1 % SDS.

An example of a first primer targeted to the oprL gene of *Pseudomonas aeruginosa* is a first primer having the base sequence given in SEQ ID NO: 4 given below, or a first primer that has the same functionality as the base sequence given in SEQ ID NO: 4 while having a base sequence consisting of at least 13 consecutive bases in a base sequence provided by the deletion, substitution, insertion, or addition of one or a plurality of bases in the base sequence described in SEQ ID NO: 4.
5'-GGTAAAGAGCGTCCGGTC-3' (SEQ ID NO: 4)

An example of a second primer targeted to the oprL gene of *Pseudomonas aeruginosa* is a second primer having the base sequence given in SEQ ID NO: 5 given below, or a second primer that has the same functionality as the base sequence given in SEQ ID NO: 5 while having a base sequence consisting of at least 13 consecutive bases in a base sequence provided by the deletion, substitution, insertion, or addition of one or a plurality of bases in the base sequence described in SEQ ID NO: 5.
5'-TTACTTCTTCAGCTCGACG-3' (SEQ ID NO: 5)

The first and second primers can specifically amplify the entire region or a partial region of a target gene, thereby enabling discrimination from another gene. Or, the amplification product from the first and second primers can be submitted to melting curve analysis and the target gene (or bacterial species containing same) can also be differentiated from another gene (or other bacterial species containing same) by comparing the melting temperature of the amplification product from the first and second primers with the melting temperature of the amplification product provided by another primer set.

The GC content of genes deriving from bacterial species other than the bacterial species that is to be differentiated - these being genes other than the gene(s) present in the bacterial species whose differentiation is being pursued and more specifically being genes other than the target gene(s) - is not particularly limited, but is preferably less than 60%.

**The** primers for amplification of a gene that is not a target gene may have any length; for example, this length can be established as appropriate in the range of approximately 10 bases to approximately 35 bases, with at least 10 bases being preferred.

The ratio for primers for amplification of a gene that is not a target gene is not particularly limited, and may be the same or may differ. When a primer set is to be used in the asymmetric PCR method, adjustment may be made so the concentration of one primer is higher than the concentration of the other primer.

Bacteria that have a GC content of less than 60% can be exemplified by *Staphylococcus epidermidis, Proteus mirabilis,* and *Acinetobacter baumannii,* but there is no limitation to these. Other examples of microorganisms that have a GC content of less than 60% are as follows: genus *Citrobacter,* such as *Citrobacter koseri, Citrobacter freundii, Citrobacter braakii,* and *Citrobacter amalonaticus;* genus *Enterobacter,* such as *Enterobacter cloacae* and *Enterobacter asburiae;* Escherichia coli; genus *Klebsiella,* such as *Klebsiella aerogenes, Klebsiella oxytoca, Klebsiella pneumoniae,* and *Klebsiella variicola; Serratia marcescens;* genus *Enterococcus,* such as *Enterococcus casseliflavus, Enterococcus faecalis,* and *Enterococcus faecium;* genus *Listeria,* such as *Listeria monocytogenes;* genus *Staphylococcus,* such as *Staphylococcus aureus* and *Staphylococcus hominis;* genus *Streptococcus,* such as *Streptococcus oralis, Streptococcus pneumoniae* and *Streptococcus pyogenes; Corynebacterium jeikeium; Neisseria meningitidis;* and genus *Candida,* such as *Candida albicans.*

The primer set for target gene amplification is not limited to the combination of a first and second primers, and may contain these first and second primers as well as other primers, for example, an additional primer, e.g., a third, fourth, fifth, and sixth primers. The detection sensitivity can be improved by the use of suitable additional primers. Such primers are presumably, for example, primers for the amplification of regions in the target gene that are different from the region that the first and second primers amplify.

The presence of a target gene, or bacterial species bearing it, contained in a sample can be detected by a nucleic acid amplification reaction using the primers described in the preceding. Target gene amplification and detection are preferably carried out in a single reaction solution from an efficiency standpoint. A label capable of specifically recognizing the amplification product may be used for detection of the amplification product after the nucleic acid amplification reaction. Such a label can be exemplified by fluorescent dyes, biotin, digoxigenin, and so forth. Either primer may be a labeled primer, but, in the case of a primer set used in asymmetric PCR, the primer with the higher molar ratio is preferably labeled. The labeling site is also not limited, but is preferably located, either directly or through a linker, at the 5'-end of the primer.

When a probe is used, a target gene can be detected by the occurrence of the formation of a complementary double-stranded gene between the probe and a single strand of the target gene that has been amplified by the asymmetric PCR. The probe is preferably designed to be complementary with the single strand of the target gene that has been amplified by the higher molar ratio primer. When fluorescence is used for the label, this fluorescence can be detected using, e.g., a fluorescence microscope, fluorescence plate reader, and so forth.

**An** example of a probe is the probe described in SEQ ID NO: 6, which captures the PCR product of *Pseudomonas aeruginosa* strain JCM14847. When the nucleic acid coding for the probe has the base sequence given in SEQ ID NO: 6, this nucleic acid may be constituted of oligonucleotide that is composed of at least 13 consecutive bases in this base sequence or in a base sequence provided by the deletion, substitution, insertion, or addition of one or a plurality of bases in the base sequence described in SEQ ID NO: 6.

**The** polynucleotide constituting, e.g., the primer, probe, and so forth, may be immobilized on, e.g., a detection substrate. The detection substrate should be able to fix and hold the polynucleotide, and various materials, e.g., glass, plastic, fiber, and so forth, can be used. A microcarrier, e.g., a microarray, is an example of the configuration of the substrate material, and a disk shape and bead shape can be considered for its shape.

In the case of a multiplex assay, the surface of the microcarrier is preferably coded with a unique identifier, e.g., a bar code. For example, the microcarrier may contain
(a) a layer that is a substantially transparent polymer layer having a first surface and a second surface, with the first and second surfaces being parallel to each other;
(b) a layer that is a substantially opaque polymer layer, wherein this substantially opaque polymer layer is affixed to the first surface of the substantially transparent polymer layer and encompasses a central region of the substantially transparent polymer layer and the substantially opaque polymer layer contains a two-dimensional shape that represents an analog code; and
(c) a capture agent for capturing an analyte, wherein the capture agent is attached to at least one of the first surface and the second surface of the substantially transparent polymer layer, in at least the central region of the substantially transparent polymer layer.

Samples providing a target gene should be samples for which the presence of the target gene, or a bacterial species containing this gene, is suspected, but are not otherwise particularly limited, and can be exemplified by bodily fluids originating from a human or nonhuman, e.g., blood, serum, plasma, urine, feces, saliva, sputum, tissue fluids, cerebrospinal fluid, and swab fluids and their dilutions, wherein blood, serum, plasma, urine, feces, and cerebrospinal fluid and their dilutions are preferred. The sample may be, for example, a food, soil, a plant, and so forth.

Besides the primer and sample, a PCR buffer containing a DNA polymerase enzyme, substrates, and magnesium ion is also added to the reaction solution.

**The** PCR may be a singleplex assay that amplifies only the target gene or may be a multiplex assay that simultaneously amplifies the target gene and one or a plurality of other genes.

Multiplex assays, e.g., multiplex PCR, are known to the individual skilled in the art. πCode technology is a multiplex assay technology: this is a technology that enables simultaneous multi-item measurement through the identification of the type of a test target based on immobilization of a probe, for measuring an antibody or a gene, on a magnetic microdisc that is imprinted on its surface with a bar code. πCode technology is disclosed in, for example, WO 2016/198954 (Japanese Translation of PCT Application No. 2018-518146) and WO 2018/052464 (Japanese Translation of PCT Application No. 2019-535003), the contents of which are incorporated in their entirety in the present Description by reference.

The present invention is specifically described using the examples provided in the following, but the present invention is not limited to or by the examples.

### Examples

### Example 1: Comparison of the results of analysis, using normal PCR and asymmetric PCR, over a substrate that uses a DNA probe directed to the Pseudomonas aeruginosa genome

### (1) Primer and probe design

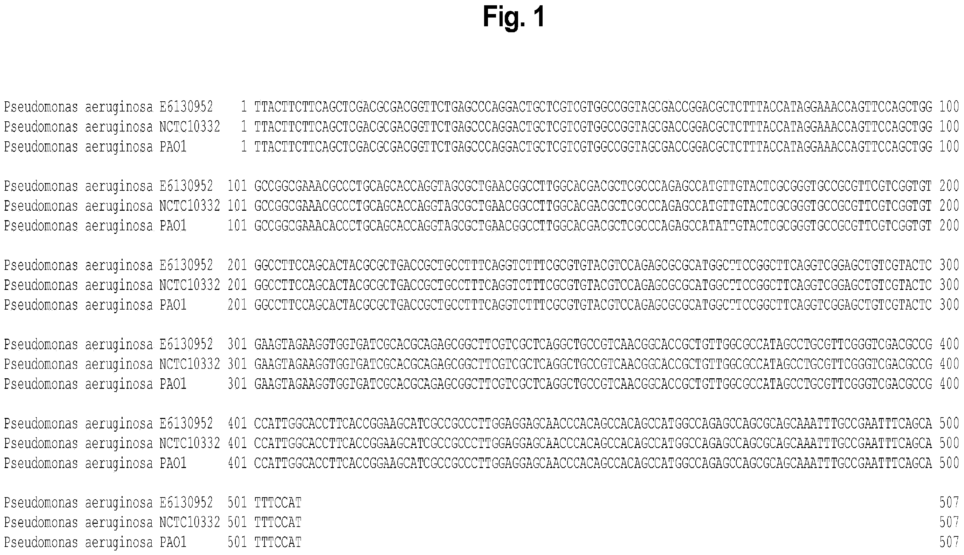

The primers given in SEQ ID NOs: 4 and 5 and the probe given in SEQ ID NO: 6 were designed based on the results, shown in Fig. 1, of multiple sequence alignment of the oprL genes (SEQ ID NOs: 1, 2, and 3) of *P. aeruginosa* strain E6130952, strain NCTC10332, and strain PAO1. The GC content of the oprL gene is 62.3%. These primers do not contain an inactivating chemical modification that is restored by the action of an activating enzyme.

### (2) Nucleic acid amplification and detection

Genomic DNA extracted from *Pseudomonas aeruginosa* strain JCM14847 was used as the template; normal PCR was carried out using a primer molar ratio between SEQ ID NO: 4 and SEQ ID NO: 5 of 1-to-1; asymmetric PCR was carried out using a primer molar ratio between SEQ ID NO: 4 and SEQ ID NO: 5 of 1-to-2.5; and an on-substrate hybridization reaction, labeling, and fluorescence measurement were carried out. A magnetic microcarrier (PlexBio Co., Ltd.) having a barcode imprinted on its surface was used as the substrate. A primer provided by biotin modification via a linker at the 5'-end of SEQ ID NO: 5 was used at this time.

A TaKaRa Multiplex Assay Kit Ver. 2 (Takara Bio Inc.) was used for amplification. In the normal PCR, each primer was added so as to provide a final concentration of 0.2 µM. In the asymmetric PCR, addition was made so the respective final concentrations were 0.2 µM and 0.5 µM. The template genomic DNA was added at 200 pg per 1 reaction. The 2x Reaction buffer in the TaKaRa Multiplex Assay Kit Ver. 2 was added to provide a final concentration of 1x, and the Multiplex Enzyme Mix was added to provide a final concentration of 1 U. A T100 Thermal Cycler (Bio-Rad Laboratories, Inc.) was used as the nucleic acid amplifying device. Using the same device, a thermal denaturation reaction was performed after the completion of the amplification reaction and before introduction to the on-substrate hybridization reaction.

The reagent used in the on-substrate hybridization reaction was prepared by mixing the following: a solution containing the substrate on which the SEQ ID NO: 6 probe, which captures the PCR product from P. aeruginosa strain JCM14847, had been immobilized, saline-sodium phosphate-EDTA buffer (SSPE buffer), and the PCR reaction solution post-thermal denaturation.

Streptavidin-phycoerythrin (PlexBio Co., Ltd.) was used in the reagent for labeling. An IntelliPlex 1000 rrCode Processor (PlexBio Co., Ltd.) was used for the device for the on-substrate hybridization reaction and labeling. A PlexBio (registered trademark) 100 Fluorescent Analyzer (PlexBio Co., Ltd.) was used as the instrument for measuring the fluorescence.

The following conditions were used.
amplification reaction conditions:
   94°C 30 seconds
   60°C 60 seconds → 94°C 30 seconds (number of cycles: 30)
   72°C 600 seconds
thermal denaturation conditions:
   95°C 5 minutes → quenching to 4°C
hybridization conditions:
   37°C incubation for 20 minutes
labeling conditions:
   37°C incubation for 10 minutes

### (3) Measurement results

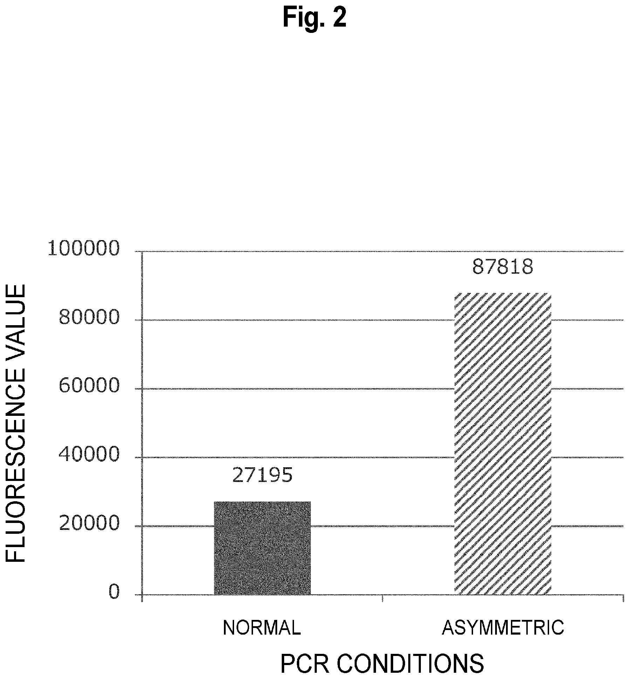

The results of the measurement are given in Fig. 2. For the use as the template of genomic DNA extracted from *P. aeruginosa* strain JCM14847, the fluorescence value provided by the asymmetric PCR was 87818, in contrast to the fluorescence value provided by the normal PCR of 27195. Based on this, it was shown that the application of asymmetric PCR to the detection of *P. aeruginosa* provided an approximately 3-fold increase in sensitivity.

### Example 2: Difference between sensitization effects for asymmetric PCR in the case of use of templates having different GC contents

### (1) Nucleic acid amplification and detection

Genomic DNA extracted from *P. aeruginosa* strain JCM14847 was used as the template; normal PCR was carried out using a primer molar ratio between SEQ ID NO: 4 and SEQ ID NO: 5 of 1-to-1; asymmetric PCR was carried out using a primer molar ratio between SEQ ID NO: 4 and SEQ ID NO: 5 of 1-to-2.5; and an on-substrate hybridization reaction, labeling, and fluorescence measurement were carried out. A primer provided by biotin modification via a linker at the 5'-end of SEQ ID NO: 5 was used at this time.

For the first comparison target, genomic DNA extracted from *Staphylococcus epidermidis* strain JCM2414 was used as the template; normal PCR was carried out using a primer molar ratio for SEQ ID NOs: 7 and 8 of 1-to-1; asymmetric PCR was carried out using a primer molar ratio between SEQ ID NO: 7 and SEQ ID NO: 8 of 1-to-2.5; and an on-substrate hybridization reaction, labeling, and fluorescence measurement were carried out. A primer provided by biotin modification via a linker at the 5'-end of SEQ ID NO: 8 was used at this time.

For the second comparison target, genomic DNA extracted from *Proteus mirabilis* strain JCM1669 was used as the template; normal PCR was carried out using a primer molar ratio for SEQ ID NOs: 10 and 11 of 1-to-1; asymmetric PCR was carried out using a primer molar ratio between SEQ ID NO: 10 and SEQ ID NO: 11 of 1-to-2.5; and an on-substrate hybridization reaction, labeling, and fluorescence measurement were carried out. A primer provided by biotin modification via a linker at the 5'-end of SEQ ID NO: 11 was used at this time.

Finally, for the third comparison target, genomic DNA extracted from *Acinetobacter baumannii* strain JCM6841 was used as the template; normal PCR was carried out using a primer molar ratio for SEQ ID NOs: 13 and 14 of 1-to-1; asymmetric PCR was carried out using a primer molar ratio for between SEQ ID NO: 13 and SEQ ID NO: 14 of 1-to-2.5; and an on-substrate hybridization reaction, labeling, and fluorescence measurement were carried out. A primer provided by biotin modification via a linker at the 5'-end of SEQ ID NO: 14 was used at this time.

A TaKaRa Multiplex Assay Kit Ver. 2 (Takara Bio Inc.) was used for amplification. In the normal PCR, each primer was added so as to provide a final concentration of 0.2 µM. In the asymmetric PCR, addition was made to provide a final concentration of 0.2 µM and a final concentration of 0.5 µM. The template genomic DNA was added at 200 pg per 1 reaction. The 2x Reaction buffer in the TaKaRa Multiplex Assay Kit Ver. 2 was added to provide a final concentration of 1x, and the Multiplex Enzyme Mix was added to provide a final concentration of 1 U. A T100 Thermal Cycler (Bio-Rad Laboratories, Inc.) was used as the nucleic acid amplifying device. Using the same device, a thermal denaturation reaction was performed after the completion of the amplification reaction and before introduction to the on-substrate hybridization reaction.

**The** reagent used in the on-substrate hybridization reaction was prepared by mixing the following: a solution containing substrate on which the probe of SEQ ID NOs: 6, 9, 12, 15 had been immobilized, saline-sodium phosphate-EDTA buffer (SSPE buffer), and the PCR reaction solution post-thermal denaturation. Here, the probe with SEQ ID NO: 9 is a probe that captures the PCR product from S. epidermidis; the probe with SEQ ID NO: 12 is a probe that captures the PCR product from *P. mirabilis;* and SEQ ID NO: 15 is a probe that captures the PCR product from *A. baumannii.*

**The** reagent used for labeling, the equipment used for the on-substrate hybridization reaction and labeling, the fluorescence measurement instrumentation, and the various conditions are the same as in Example 1.

### (2) Measurement results

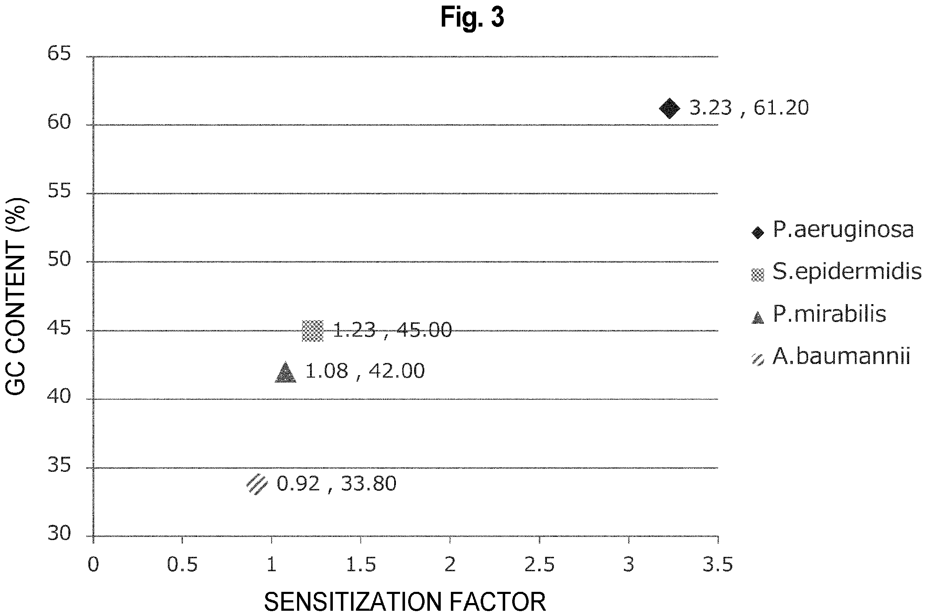

Fig. 3 shows a plot of the results using the sensitization factor - which is the value provided by dividing the fluorescence value in the asymmetric PCR by the fluorescence value in the normal PCR - for the horizontal axis, and using the GC content of the PCR product for each item, represented by SEQ ID NOs: 16, 17, 18, and 19, for the vertical axis. Here, the sensitization factor refers to the numerical value provided by dividing the fluorescence value yielded by execution of the amplification reaction using asymmetric PCR and carrying out detection on the resulting amplification product, by the fluorescence value yielded by execution of the amplification reaction using normal PCR and carrying out detection on the resulting amplification product; a higher sensitization factor means a higher detection sensitivity over normal PCR. The following is shown based on the results in Fig. 3: for the case of a low GC content, the sensitization factor increases in accordance with the GC content; when the GC content of the target gene is at least 60%, there is a very substantial increase in the sensitization factor, i.e., the detection sensitivity.

**As** shown in Fig. 3, a positive correlation between the sensitization factor and GC content was confirmed. With regard to the reason for the appearance of this correlation, it is thought that the rate of a nucleic acid hybridization reaction exhibits a large dependence on the composition of the nucleic acid. The following model has been proposed for the hybridization reaction: some of the complementary bases repeatedly approach and separate between two molecules that have complementary sequences, and, when association with a completely complementary sequence eventually occurs, base pairs are formed that will close the zipper from there. It has been reported, according to results calculated using an 8-base model sequence, that the rate of base pair formation for a high-GC base sequence has been calculated to be 3.2-times that for a high-AT base sequence, and that a high-GC base sequence more rapidly associates with its complementary sequence and forms a complementary strand (DNA hybridization kinetics: zippering, internal displacement and sequence dependence. Nucleic Acids Res. 2013 Oct; 41 (19) : 8886-8895).

The targets for on-substrate capture in this example are PCR products provided by amplification as double-stranded DNA and denaturation. Thus, it is thought that for the PCR product the hybridization reaction proceeds in a mode in which formation of a partial complementary strand with the on-substrate probe coexists with complementary strand formation with PCR product. The following is hypothesized based on the aforementioned measurement results: as the GC content of the product increases, the capture reaction by the probe, as well as PCR product-to-PCR product complementary strand formation, are both accelerated, and, while the capture reaction by the probe did not proceed satisfactorily in the normal PCR, the capture reaction by the probe proceeded effectively due to the excess production, through the application of asymmetric PCR, of molecules that are captured onto the substrate and a remarkable sensitizing effect was obtained.

With the low GC content *A*. *baumannii,* on the other hand, it was found that the fluorescence value was reduced, with a sensitization factor of 0.92, when asymmetric PCR was run. It is hypothesized, with regard to on-substrate detection, that two types of hybridization reactions are occurring - in the mode in which partial complementary chain formation with the probe already on the substrate coexists with complementary chain formation with PCR product - and in addition that numerous parameters, e.g., the number of molecules of capture targets, GC content, base pair formation rate, and so forth, are related and are reflected in the final fluorescence value. It is known that the PCR product yield in asymmetric PCR is lower than in normal PCR, and the present results show that asymmetric PCR can act unfavorably with low GC content target genes.

Based on the preceding, it has been demonstrated that the detection sensitivity is improved by the application of asymmetric PCR to the detection of a template having a GC content of at least 60%.

### Example 3: Example of simultaneous detection of a gene having a GC content of at least 60% and a gene without this property

### (1) Nucleic acid amplification and detection

A sample 1 was prepared which was confirmed, by a culture and identification procedure, to contain two bacterial species, i.e., *Pseudomonas aeruginosa* (GC content of strain PA01: 66.6%) and *Enterococcus casseliflavus.* It is known that the GC content of *P. aeruginosa* strain PA01 is 66.6% and the GC content for *Enterococcus casseliflavus* is 42.9% (Sanderson, H., Ortega-Polo, R., Zaheer, R. et al., Comparative genomics of multidrug-resistant Enterococcus spp. isolated from wastewater treatment plants. BMC Microbiol 20, 20 (2020). https://doi.org)/10.1186/s 12866-019-1683-4).

Amplification by multiplex PCR was carried out using as template the genomic DNA extracted from the sample using a QIAamp BiOstic Bacteremia DNA Kit (QIAGEN) and using the standard protocol provided with the reagent, and an on-substrate hybridization reaction, labeling, and fluorescence measurement were also carried out. As in Examples 1 and 2, a magnetic microcarrier (PlexBio Co., Ltd.) having a barcode imprinted on its surface was used as the substrate. In addition, the reagent used for labeling, the on-substrate hybridization reaction, the instrument used for labeling, the fluorescence measurement instrument, and the other conditions are the same as in Examples 1 and 2, unless specifically indicated otherwise.

A TaKaRa Multiplex Assay Kit Ver. 2 (Takara Bio Inc.) was used for amplification. With regard to the primers, the primers given by SEQ ID NOs: 4 and 5 were used for *Pseudomonas aeruginosa,* and the primers given by SEQ ID NOs: 20, 21, 22, and 23 were used for *Enterococcus casseliflavus.* In the normal PCR, each primer was added so as to provide a final concentration of 0.2 µM for all the primers. In the asymmetric PCR, addition was made so the final concentration for the primer other than SEQ ID NO: 5 was 0.2 µM and addition was made so the primer with SEQ ID NO: 5 had a final concentration of 0.5 µM. Primers provided by biotin modification via a linker at the 5'-end of SEQ ID NOs: 5 and 23 were used. The template solution was added at 5 µL per 1 reaction. The 2x Reaction buffer in the TaKaRa Multiplex Assay Kit Ver. 2 was added to provide a final concentration of 1x, and the Multiplex Enzyme Mix was added to provide a final concentration of 1 U. A T100 Thermal Cycler (Bio-Rad Laboratories, Inc.) was used as the nucleic acid amplifying device. Using the same device, a thermal denaturation reaction was performed after the completion of the amplification reaction and before introduction to the on-substrate hybridization reaction.

**The** reagent used in the on-substrate hybridization reaction was prepared by mixing the following: a solution containing a substrate 1 on which a probe (SEQ ID NO: 6) that captured Pseudomonas aeruginosa had been immobilized, and containing a substrate 2 on which a probe (SEQ ID NO: 24) that captured *Enterococcus casseliflavus* had been immobilized; saline-sodium phosphate-EDTA buffer (SSPE buffer); and the PCR reaction solution post-thermal denaturation.

For substrate 1, on which a probe that captured *Pseudomonas aeruginosa* had been immobilized, the fluorescence values provided by subtracting the blank were 1622 for the normal PCR and 13731 for the use of asymmetric PCR, and the detection sensitivity was thus substantially improved. For substrate 2, on which a probe that captured *Enterococcus casseliflavus* had been immobilized, the detection sensitivity was also satisfactorily high for the normal PCR, while a negative effect was also not exercised on the results for substrate 1 for the amplification of *Pseudomonas aeruginosa* by asymmetric PCR, and the detection sensitivity was also raised according to the results for substrate 2.

Thus, asymmetric PCR also worked effectively for the simultaneous detection of a gene having a GC content of at least 60% and a gene without this property, and the efficacy of the present invention could be confirmed.

## Claims

1. A method for amplifying, in a reaction solution, at least one target gene having a GC content of at least 60%, the method comprising:
a step of amplifying a specific base sequence in a target gene by using a primer set consisting of a first primer and a second primer for target gene amplification,
wherein one primer in the primer set is added to the reaction solution at a molar ratio higher than the other primer and neither primer is a hot start primer that contains an inactivating chemical modification that is restored by an action of an activating enzyme.

2. The method according to claim 1, wherein the reaction solution contains one or a plurality of genes that are not a target gene and wherein the method comprises simultaneous amplification of both a target gene and the one or a plurality of genes that are not a target gene.

3. The method according to claim 1 or 2, wherein the molar ratio of the primers in the primer set is 1 : 1.5 to 20.0.

4. The method according to any one of claims 1 to 3, wherein a target gene is a base sequence consisting of at least 13 consecutive bases in the genomic sequence of *Pseudomonas aeruginosa.*

5. The method according to any one of claims 1 to 4, wherein the first primer is a primer that hybridizes, under stringent conditions, with a base sequence described in any of SEQ ID NOs: 1 to 3 or with a base sequence provided by deletion, substitution, insertion, or addition of one or a plurality of bases in a base sequence described in any of SEQ ID NOs: 1 to 3, and the second primer is a primer that hybridizes, under stringent conditions, with a complementary strand sequence for a base sequence described in any of SEQ ID NOs: 1 to 3 or with a base sequence provided by the deletion, substitution, insertion, or addition of one or a plurality of bases in a complementary strand sequence for a base sequence described in any of SEQ ID NOs: 1 to 3.

6. The method according to any one of claims 1 to 5, wherein the first primer is an oligonucleotide consisting of at least 13 consecutive bases in the base sequence described in SEQ ID NO: 4 or in a base sequence provided by the deletion, substitution, insertion, or addition of one or a plurality of bases in the base sequence described in SEQ ID NO: 4.

7. The method according to any one of claims 1 to 6, wherein the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.

8. The method according to any one of claims 1 to 7, wherein a primer is labeled with biotin.

9. The method according to any one of claims 1 to 8, wherein the primer with a higher molar ratio is labeled with biotin.

10. A method of detecting, in one reaction solution, at least one target gene having a GC content greater than 60%, the method comprising:
a step of detecting a target gene by using a probe that is complementary to a single strand of a target gene that has been amplified by the method described in any one of claims 1 to 9.

11. The method according to claim 10, wherein the probe is complementary to a single strand of the target gene that has been amplified by the primer with the higher molar ratio.

12. The method according to claim 10 or 11, wherein a target gene is detected through occurrence of complementary double-stranded gene formation from the probe and a single strand of the target gene.

13. The method according to any one of claims 1 to 12, wherein a target gene originates from *Pseudomonas aeruginosa* and a bacterial gene from other than *Pseudomonas aeruginosa* is differentiated from *Pseudomonas aeruginosa.*

14. The method according to any one of claims 10 to 13, wherein the probe is immobilized on a detection substrate material.

15. The method according to any one of claims 10 to 14, wherein the probe is labeled.

16. The method according to any one of claims 10 to 15, wherein a probe nucleic acid comprises an oligonucleotide consisting of at least 13 consecutive bases in the base sequence described in SEQ ID NO: 6 or in a base sequence provided by the deletion, substitution, insertion, or addition of one or a plurality of bases in the base sequence described in SEQ ID NO: 6.

17. The method according to any one of claims 14 to 16, wherein a detection substrate material is a coded microcarrier.

18. The method according to claim 17, wherein the microcarrier comprises:
(a) a layer that is a substantially transparent polymer layer having a first surface and a second surface, with the first and second surfaces being parallel to each other;
(b) a layer that is a substantially opaque polymer layer, this substantially opaque polymer layer being affixed to the first surface of the substantially transparent polymer layer and encompassing a central region of the substantially transparent polymer layer, and the substantially opaque polymer layer containing a two-dimensional shape that represents an analog code; and
(c) a capture agent for capturing an analyte, the capture agent being attached to at least one of the first surface and the second surface of the substantially transparent polymer layer, in at least the central region of the substantially transparent polymer layer.

19. A primer set, consisting of a first primer and a second primer, for detecting the presence of at least one target gene having a GC content of at least 60%, wherein
the first primer is an oligonucleotide consisting of at least 13 consecutive bases in the base sequence described in SEQ ID NO: 4 or in a base sequence provided by deletion, substitution, insertion, or addition of one or a plurality of bases in the base sequence described in SEQ ID NO: 4, and
the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.

20. The primer set according to claim 19, wherein a target gene is a base sequence consisting of at least 13 consecutive bases in the genomic sequence of Pseudomonas aeruginosa.

21. The primer set according to claim 19 or 20, wherein the first primer is a primer that hybridizes, under stringent conditions, with a base sequence described in any of SEQ ID NOs: 1 to 3 or with a base sequence provided by the deletion, substitution, insertion, or addition of one or a plurality of bases in a base sequence described in any of SEQ ID NOs: 1 to 3, and the second primer is a primer that hybridizes, under stringent conditions, with a complementary strand sequence for a base sequence described in any of SEQ ID NOs: 1 to 3 or with a base sequence provided by the deletion, substitution, insertion, or addition of one or a plurality of bases in a complementary strand sequence for a base sequence described in any of SEQ ID NOs: 1 to 3.

22. The primer set according to any one of claims 19 to 21, wherein
the first primer is an oligonucleotide consisting of at least 13 consecutive bases in the base sequence described in SEQ ID NO: 4 or in a base sequence provided by the deletion, substitution, insertion, or addition of one or a plurality of bases in the base sequence described in SEQ ID NO: 4.

23. The primer set according to any one of claims 19 to 22, wherein
the second primer is an oligonucleotide consisting of at least 13 consecutive bases in the base sequence described in SEQ ID NO: 5 or in a base sequence provided by the deletion, substitution, insertion, or addition of one or a plurality of bases in the base sequence described in SEQ ID NO: 5.
